# EUROPEAN PATENT SPECIFICATION

(11) **EP 3 773 380 B1**
(45) Date of publication and mention of the grant of the patent: **22.03.2023**
(21) Application number: 19712669.1
(22) Date of filing: 02.04.2019
(51) Int. Cl.: A61N 5/06, A61F 13/00, A61F 13/02, A61F 13/12, A61B 90/00, A61F 7/00, A61F 7/02, A61F 9/04

(54) **A SYSTEM FOR THE TREATMENT OF DRY EYE SYNDROME**
SYSTEM ZUR BEHANDLUNG DES TROCKENEN-AUGEN-SYNDROMS
SYSTÈME DE TRAITEMENT DU SYNDROME DE SÉCHERESSE OCULAIRE

(30) Priority: 04.04.2018 EP 18165650
(43) Date of publication of application: 17.02.2021
(73) Proprietor: Alvalux Medical, 4000 Liège (BE)
(72) Inventor: ALVAREZ, Michel, 4000 Liège (BE); ANDRE, Nicolas, 4000 Liège (BE)
(74) Representative: Gevers Patents
(86) International application number: PCT/EP2019/058282
(87) International publication number: WO 2019/193000

(56) References cited:
- WO-A1-2015/024076
- WO-A1-2017/097708
- US-A- 6 034 293
- US-A1- 2016 120 693
- US-A1- 2017 087 009

## Description

### FIELD OF THE INVENTION

The present invention relates to a system for the treatment of dry eye syndrome but is not exclusively related thereto.

### BACKGROUND OF THE INVENTION

Meibomian Gland Dysfunction (MGD) is a chronic, progressive disease and the leading cause of Dry Eye Syndrome (DES). It was estimated that nearly 337 million people were suffering from severe, moderate, or episodic DES worldwide in 2015. Up to 86% of all dry eye sufferers have MGD. The prevalence of MGD in several large general Asian population-based studies has been found to be as high as 69%. Other sources have estimated a widespread prevalence of between 39 to 50% of the population that increases with age, and 40% of these suffer from moderate to severe forms of DES. In the contact lens wearing population, 50 to 75% suffer from dry eye symptoms. MGD is one of the most common diseases observed in clinics accounting for about one-fifth of all visits to eye care professionals.

Meibomian glands are essential in keeping the ocular surface (including the sensitive cornea) clean, healthy and well-lubricated, and, the number of such glands in the upper eye lid is twice that of those in the lower lid. Meibomian glands produce meibum, an essential oily substance of tear film. Dysfunction of these glands is a common disorder, and, can be characterized by alterations in gland morphology and location, as well as a waning in quality and quantity of gland secretion. Symptoms include irritation, burning, itchiness, redness, pain, ocular fatigue and visual disturbance.

A contributor of DES and corneal problems (including exposure, keratopathy, ulceration and keratitis) is Lagophthalmos (LGT). LGT is a condition present in up to 20% of the population. LGT is the incomplete or defective closure of the eyelids, and, the inability to blink and effectively close the eyes leads to corneal exposure and excessive evaporation of the tear film leading to DES. Correct and timely diagnosis allows greater opportunity for relief of patient suffering and prevention of severe ocular surface pathology. The main cause of LGT is facial nerve paralysis (paralytic LGT), but it also occurs after trauma or surgery (cicatricial LGT) or during sleep (nocturnal LGT).

Nocturnal LGT is the inability to close the eyelids during sleep. Dry eyes could exacerbate the symptoms of nocturnal LGT, and nocturnal LGT can also worsen dry eyes with excessive exposure during sleep. Blepharitis and MGD will further compromise the tear film and worsen the LGT. Nocturnal LGT is a relatively common disorder and a potential cause of previously undiagnosed Chronic Keratitis (CK). Patients typically present with their symptoms early in the day within a few hours of waking, and, are more pronounced immediately after waking. The symptoms may comprise one or more of soreness, dryness, foreign body sensation or sharp pain in one or both eyes, often associated with watering and sometimes blurred vision. Some patients will present redness of the eye. If damage from exposure is sufficiently severe, it may not heal during the waking hours. In cases of advanced keratopathy and corneal ulceration, the symptoms and presentation may be severe.

LGT patients may also suffer from poor sleep quality which also exacerbates other health problems and reduces overall well-being. The added level of dryness and increased frequency of blepharitis with poorer quality tears in middle-aged and older adults may account for the higher incidence of symptomatic nocturnal LGT in older patients. Taping the eyelid closed with medical paper tape during sleep has proven to be helpful for some patients but some patients may not tolerate the tape on their periorbital skin because of sensitivity to the tape adhesives; while some female patients may not comply with this treatment because they worry about pulling away eyelashes while removing the tape. Therefore, there is an unmet need to improve methods and devices in this area.

Besides taping the eyelid closed, medical treatment and supportive care for LGT implicated corneal exposure is similar to standard DES care. Treatment includes the use of eyedrops (artificial tears) administered frequently (at least four times per day) in order to supplement the patient's tear film. However, eyedrops cannot be applied when sleeping. Ointments can be applied to the cornea once at bedtime or throughout the day in cases of severe corneal exposure. Moisture goggles also may be used. Infectious corneal ulcers should be treated with an appropriate antibiotic therapy, and, it may be necessary to patch the eye closed or place a Frost suture for temporary protection of the cornea. Generally, a combination of these standard DES therapies along with eyelid taping or eyelid weights get a better and faster response for LGT, but, again, many limitations remain.

Given the increased recognition of the importance of MGD, a great amount of attention has been paid to therapies targeting this condition and DES overall. Typical DES treatments include using topical pharmaceutical agents in the form of eyedrops and ointments which coat the eyes. However, these artificial tears, gels, and ointments may be effective in treating DES symptoms but not the underlying cause in the case of MGD, that is, the release of natural meibum from the glands. Moreover, treatment for dry eyes can include a short course of topical corticosteroid eyedrops; however, this is not a good long-term measure owing to potential side effects of increased intraocular pressure and cataract formation. Lastly, these agents do not aid in the sealing or complete closure of the eyelid in the case of LGT sufferers.

In more severe cases of DES, invasive treatments include inserts such as punctal plugs which plug the patient's tear ducts to prevent tear film from draining away too rapidly from the surface of the eye or ocular inserts such as LACRISERT^{®} (from Merck & Co., Inc, N.J.). These foreign bodies placed in eye can be expelled, cause infection, discomfort, and other complications. Moreover, they do not address the issues associated with LGT.

Common home-based treatments for MGD rely on warm compresses and lid hygiene for freeing obstructed meibum, and, may often be accompanied with antibiotics and anti-inflammatory agents to improve the quality of the meibum.

However, each of these treatments may have a different shortcoming and the treatment of MGD remains challenging.

Although effective, current methods for heat therapy (including warm compresses) of the meibum glands have various limitations, dangers, and unmet needs. The challenge with any form of front surface eyelid heating is to be able to transfer therapeutic levels of heat to the meibomian glands, typically at temperatures of at least 40°C (104°F), while not risking thermal injury to the ocular surface or the skin.

Approaches using a warm compress, including eye masks and moist towels heated by microwave, tend to provide varying temperature regions on the surface of these devices including dangerously high temperatures predisposing the user skin to unwanted burns and discomfort. The most recommended method for warm compress wet towel is the 'bundle' method, where the temperature of the wet 'bundled' cloth after being microwave measured to be anywhere between 49 to 77°C, whereas the target surface temperature to achieve is 45°C, and, therefore this tends to be a very imprecise method for a user.

Another device MeiboPatch^{®} dry eye mask (from NicOx SA, a French pharmaceutical company) may have an extreme 20°C temp difference between the center point (for example, at 59.3°C) and the periphery (for example, at 40.7°C) of device skin contact area, and, this tends to be after rigorous shaking of its internal grape seeds to allow for better heat distribution following heating in a microwave. Moreover, these devices tend to suffer from a rapid cooling curve, and hence constantly changing temperature, and thus require repeated handling and microwaving for continued use.

Non-microwave heat methods include the EyeGiene^{®} dry eye mask (from Eyedetec Medical, a US company headquartered in Northern California) which uses exothermic oxidation of iron contained in disposable inserts to generate heat. Whilst the heat profile appears to show better uniformity than MeiboPatch and other microwave approaches, limitations of the EyeGiene dry eye mask include one-time use with repeated waste and costs for the disposable insert refills, it tends not to be suitable for therapy during sleep as the one-time inserts cool within 15 minutes, and, the one size mask tends not to contour to the eyelids thus varying the amount and distribution of heat to the meibomian glands. Moreover, the 'one size' mask also tends to cause excessive pressure on eyeball, which can create elevated intraocular pressure, a risk factor for glaucoma. Thus, it tends not to be recommended nor comfortably tolerable for multiple daily sessions or overnight wear, and, as it is a full mask, it cannot be used one eye at a time.

Other approaches include moisture retention goggles, such as, EyeSeals^{®} (from Eye Eco Inc.), but patients report concerns over fit, for example, the goggles may be too large for some users, particularly, ladies with small or narrow faces, with varying anatomical difference in the upper face including nose bridge and eye socket differences among humans, and, like masks, multi-hour fit and comfort of these devices are a concern. Goggles, by design, create limited, and therefore, excessive pressure points at the perimeter seals and nose bridge areas where the pressure is concentrated, and result in pressure marks due to the viscoelasticity of skin, in a similar way to wearing swim goggles all night. Moreover, goggle approaches tend not to directly treat meibomian glands for MGD or shut the eyelids for LGT.

The preceding examples are not self-powered or programmed 'energized' devices. They do not provide for controlled temperature, cyclical, hands-free, overnight therapy.

More innovative and recent "energized" face mask devices, such as, the Eye Massager HQ-365 (available from many suppliers), tend to use infrared or thermal energy to deliver heat to the eyes; however, these 'one size' masks have the same issues as non-energized masks with respect to varying facial geometry amongst humans and with the accompanying tendency to deliver varying energy levels to the eye area depending on fit and distance from the heat element to the eyelid. These bulky and heavy devices cover a large area of the face making them often uncomfortable and only useful for single short sessions, and tend not to recommended for overnight or cyclical therapy use, especially as they generally have bulky cables and connections. Equally concerning is that they tend not provide a direct, conforming, adhesive, skin contact with eyelid tissue, and therefore, do not 'extend' the meibomian glands fully in the upper eyelid to provide a more uniform and proximal heat therapy; nor do they seal the eyelid shut to address the needs and comfort of LGT sufferers.

Lastly, new, invasive, 'energized' treatments for DES requiring doctor intervention and in-clinic surgical procedures include Lipiflow^{®} (from a Johnson & Johnson company) and OcuLeve^{®} (from Oculeve, Inc.) respectively. These tend to be inconvenient, expensive approaches and fall into category of therapy reserved for the most severe cases of DES and MGD. Moreover, they tend not to seal the eyelid shut for treating LGT with night therapy.

Accordingly, there exists a need for methods and devices which are easy to use at home; can operate overnight for maximum patient convenience while they sleep; can treat multiple conditions and disorders simultaneously; are non-invasive; conform to the patient's unique eye and face geometry; are reversible/removable at will; allow one eye to be treated at a time for maximum productivity and day use; are ambulatory (i.e. device does not need to be plugged into wall to operate, or have cables around their body limiting motion); uses safe, rechargeable power sources and low energy levels; have smart, programmed micro-controlled temperature; offer multiple cyclical sessions with one wear and one charge including convenient overnight therapy; seals the eyelid gently for LGT sufferers without pressure on eyeball for those at risk of or suffering from glaucoma; and are non-bulky and light (unlike heavy 'energized' masks), and comfortable (i.e. no pressure points from seals as in goggles or large masks covering much of your face unnecessarily) etc.

Document US 2017 087 009 shows a patch for meibomian glands warming. The patch can be attached over or under the eye with an adhesive. The patch may emit RF, IR or visible light to warm the glands. A heat reflective layer may be present as well.

Document US 2016 120 693 shows another patch for meibomian glands warming. The patch can be attached to an eyelid when the eye is closed with an adhesive layer. There is a chemical heat source and a heat reflecting layer.

Document WO 2015 024 076 shows a patch applied over the closed eye to protect it during a surgical procedure. The patch as a layer of a weaker adhesive to be placed over the closed eyelid and an outer ring of a stronger adhesive that, in use, attaches to the skin around the eye.

### SUMMARY OF THE INVENTION

The invention is defined in the following claims. Other embodiments, examples and, in particular, methods are not a part of the invention.

There is disclosed an eye patch for treating eye disorders, the eye patch comprising:
a first adhesive layer configured for adhering an inner area of the eye patch to secure an eyelid of a user closed, the first adhesive layer comprising a silicone adhesive contact layer having a first coat formula;
a second adhesive layer configured for adhering an outer area of the eye patch to an area around an eye of the user, the second adhesive layer comprising a silicone adhesive contact layer having a second coat formula,
the second coat formula being of stronger adherence than the first coat formula of the first adhesive layer, the second adhesive area having first and second portions where the second portion is configured to surround the first portion and to be attached to the area around the eye of the user;
a reflecting layer adhered to the first portion of the second adhesive layer, the reflecting layer being configured for reflecting radiation towards the eyelid of a user;
a flexible element positioned in the inner area of the eye patch between the first layer and the first adhesive layer and configured for providing radiation therapy in accordance with a predetermined radiation therapy program; and
a connection configured for connecting the flexible element to a control unit and for providing electrical power to the flexible element in accordance with the predetermined radiation therapy.

In effect, the therapy system comprises an energized headband with a flexible dual adhesive arrangement which can be used with 'single or dual' eye patches.

By providing a small, light, wearable system which can easily conform and stick gently to the eyelid, and simultaneously 'seal and extend' the eyelid, for the best position and uniformity of therapy to direct and distribute therapeutic radiation onto the underlying meibomian glands while alleviating LGT, the benefits of a combined smart headband and hybrid seal thermal energy patches can readily be realized without the disadvantages of the prior art systems. In particular, due to a dual adhesive design, a triple mode therapy comprising IR/thermal and LLLT, silicone compression with hydration, and eyelid sealing and extending, the wearable system offers health and well-being improvement for DES/MGD/CK/LGT/ sufferers.

The system comprises a control unit which is mountable in a soft wearable headband and is connected to one or two eye patches to provide radiant energy thereto. A microcontroller unit (MCU) is mounted on a printed circuit board (PCB), and, controls the supply of radiant energy to each eye patch, either continuously for a predetermined time or periodically in accordance with a predetermined program. A battery is mounted within the control unit on the PCB and powers components in the control unit, for example, the MCU.

The system has the advantage of being able to provide the following:
1. Night-time relief to avoid waking with bad symptoms like sealed/crusted lids or `sandpaper' eyes:
   - most solutions only work one cycle while you are awake, typically for a 10 to 15 minute session, and you should not fall asleep with them; others are non-energized, and provide non-programmed continuous therapy like moisture goggles or tapes that do not heat to loosen or free the meibum and address the issues of MGD

   The disclosed system provides 'cyclical therapy' which provides multicycle therapy while sleeping and mitigates eyes drying out overnight so that a user wakes with fresh, healthy eyes. The system works to release trapped or clogged meibum due to MGD more naturally whilst also treating LGT and DES.
2. Universal "sizing" or best custom fit that avoid excessive pressure points, seals moisture, and limits light entry:
   - Bad fit and pressure points are common complaints of eye goggle and eye mask wearers with the goggles and masks being too big for some and too small for other making the fit difficult to get right particularly around the nose bridge area

   The present disclosure provides 'press-on' energized, high-flex, eye patches with a "dual adhesion system" allowing gentle adhesion on sensitive eyelid tissue and more robust adhesion on peripheral skin. This allows personalized custom fit to each individual eyelid and eye area with higher comfort and safety when removing patch. In particular, silicone adhesive (SA) multilayer patch technology is implemented with light blocking and IR reflecting fabric liner which seals in moisture, enhances IR thermal therapy, and seals eyelid for LGT.
3. Keeping eyelids closed (up to 20% of people suffer from lagophthalmos):
   - For nights, in severe cases the 'default' may be medical tape, possibly even medical tape PLUS a moisture goggle, but both tape and goggles have their issues.

   The eye patches in the system provide a gentle personalized fit to keep eyelids closed with the silicone trapping moisture but allows skin to breathe.
4. Preventing excessive pressure on eyeball with resulting high intraocular pressure (IOP) and glaucoma risk:
   - The EyeGiene mask and other face masks tend to press hard on the eye offering little conformity to account for the wide variations in anatomic contour and shape of nose and eye region from person to person

   The infrared (IR) heating is gently applied 'against' the eyelid, and soft silicone eye patch adhesive allows the perfect conformance with each eyelid and peri-orbital region without any excessive pressure being placed on eye preventing high IOP and optic nerve damage/glaucoma risk etc.
5. Deliver cyclical treatment, not just single session therapy like heated damp washcloth, microwave mask, or other non-energized heat generating masks:
   By providing a rechargeable and microcontroller programmable control unit centered above nose on forehead in headband, cyclical on/off heat therapy is provided through the patches all night during sleep. A larger patch embodiment can provide LLLT (e.g. red light therapy) and improved hydration for improving periorbital wrinkles and related aesthetic issues as well as providing therapy to address eye disorders as described above.
6. Improved temperature control:
   - no "hot spots" from microwave heating as shown with various eye masks and towel compresses which can burn skin and cornea

   The MCU controls radiant energy delivery, both thermal and optical, for enhanced safety and comfort together with the use of improved design of the heating coil. Added IR reflecting woven liner and/or graphene nanolayers can increase and improve planar heat distribution to eyelid area.
7. Sleeping position:
   - Ability to sleep on side of face as bulky masks or goggles offer limited head positions for sleep

   An ultra slim headband design and flat on eye patches, of the disclosed system have no protrusions beside a small central forehead bump (approximately 40mm only) within headband that houses the control unit thus offering the lowest profile wearable around the eyes and highest degree of freedom for head on pillow positions during sleep.
8. One eye:
   - Ability to treat only one eye at a time for daytime freedom of mobility and productivity (work or pleasure)

   By having a roll-up eye patch hidden in headband of the system when not in use, a single eye patch can be applied to one eye as necessary. Appropriate settings for one or both eyes is possible with the MCU, for example, when only one eye patch is used, the other eye is free for working at desk or doing chores around the home etc.
9. Economy:
   - Washable / multi-use elements

   The control unit of the system is rechargeable and reusable for at least 1 year. The headband is washable, and the eye patches are washable after each use and disposable after approximately up to 20 uses due to varying patient skin conditions (e.g. excessive perspiration or oils), and, the eventual weakening of the SA layer if used following instructions for use (i.e. placing eye patch on clean, dry skin).
10. Energy efficiency:
   - Energized devices tend to require lots of batteries which is not eco-friendly The control unit is rechargeable via standard micro-USB port.
11. Freedom to move around:
   - Cable-free wear needed to prevent tangling with arms, legs, etc.

   No cables are required which need to be attached to power outlets or across body during therapy or wear. In particular, there is no tangling during sleep, and, all elements of system are contained within upper face and forehead areas in the headband.
12. Weight:
   - Not heavy nor bulky system needed which also does not cover much of face as with many energized masks or goggles with large cords and cables

   By having a limited bulk headband housing the control unit and very light eye patches, the system can be imperceptible to the user. Typically, the control unit weighs under 10g and is up to 14mm thick with a 10cm² footprint and its weight is effectively unnoticeable.
13. Breathability:
   - Non-breathable, over-hydrating, skin contact material may increase the risk of excessive skin perspiration and rash in use

   The silicone material of which the eye patch skin contact layer is made is breathable but hydrophobic and helps hydrate skin.
14. Integrity:
   - Polyurethane (PU) used for skin contact foam in many other masks and goggle liners tends to disintegrate with repeated use.

   The eye patches of the present disclosure are made from silicone as a preferred, skin friendly material which is found in the higher priced goggles/masks.
15. Ability to make appropriate contact:
   - Contact vs non-contact with eyelid is important to achieve the benefits of low-power requirements and proper heating of eyelid and meibomian glands while also sealing the eyelid for LGT

   Eye patches of the present disclosure provide a 'light' custom fit contact with upper (and lower) eyelid. This simultaneously seals and extends the upper eyelid, in particular, where most of the meibum glands are located, thereby allowing the meibomian glands to be in their fullest 'extended' state for a more optimal radiant therapy.
16. Personalization:
   - Personalized treatment options preferred and not a "one solution fits all"

   The control unit may have a number of pre-programmed cycle options for low-medium-high power therapy and single or dual eye mode settings.
17. Check wear time etc.:
   - Tracking / training to help user adherence to therapy.

Biosensors are provided in the control unit to detect wear time. Such sensors may include a capacitive touch and/or temperature sensor element which detects and records when control unit is active on a user compared to being on the shelf.

### BRIEF DESCRIPTION OF THE DRAWINGS

Fig. 1 is a schematic illustration of a first embodiment of a therapy system in accordance with the present disclosure;
Fig. 2 is a schematic illustration of a second embodiment of the therapy system in accordance with the present disclosure;
Figs. 3a to 3e are schematic views of a control unit for use in the therapy systems of Figs. 1 and 2;
Fig. 4 is a schematic illustration of a top view of the control unit of Figs. 3a to 3e with its upper casing removed;
Fig. 5 is a schematic illustration of the layers of an eye patch for use in the therapy systems of Figs. 1 and 2;
Figs. 6a to 6c are schematic illustrations of different eye patch configurations with and without light pipes;
Fig. 7 is similar to Fig. 6b but including 'branched' light pipes to focus light in specific areas;
Fig. 8 is similar to Fig. 6b but including additional ultra-thin light-emitting diodes (LEDs) to focus light in specific areas;
Figs. 9a and 9b are schematic illustrations of the positioning of lacrimal glands and meibomian glands in an eye;
Fig. 9c is a schematic illustration of an eye patch positioned on an eye over an eyelid;
Figs. 10a, 10b and 10c are schematic illustrations of top, side, exploded and bottom perspective views respectively of a headband for the therapy system as shown in Figs. 1 and 2;
Figs. 11a to 11f illustrate view of a user wearing the system in accordance with the present disclosure; and
Fig. 12 illustrates a flowchart showing steps for using the therapy system in accordance with the present disclosure.

### DETAILED DESCRIPTION OF THE INVENTION

The present disclosure relates to devices for treatment of dry eye syndrome (DES) and chronic keratitis (CK), and, particularly, the combined treatment of meibomian gland dysfunction (MGD) and lagophthalmos (LGT), and other related conditions. More particularly, it relates to devices for single or dual eye simultaneous treatment of DES/CK/MGD/LGT using re-usable adhesive, micro-controlled, cyclical thermal energy eye patches which are specifically "hybrid designed" for gentle adherence to, and closure of, eyelid skin in order to close or shut fully an eye of a patient or user for treating LGT. At the same time, the eye patches ensure a more complete extension of' the meibomian glands to improve exposure to thermal energy therapy, as well as, a more robust adherence to peripheral skin around the eye region. The therapy system includes a 'non-mask' headband containing a rechargeable control unit which delivers programmed electrical (and photonic) energy delivery to connected eye patches.

Each eye patch has a dual adhesion system where different areas or regions of the eye patch has a different adherence. The dual adhesion system comprises a first adhesive layer which is designed to secure an eyelid of a user in a closed position so that the meibomian glands, particularly, those in the upper eyelid, are readily accessible and not partially or fully retracted under the orbital fat layer as with a partially or fully open eyelid, and, a second adhesive layer which is designed to secure the periphery of the eye patch to skin of a user around the eyelid area. Depending on the size of the eye patch, the second adhesive layer may adhere to skin within the eye socket or surrounding the eyelid, to skin outside of the eye socket, or to skin over the forehead etc. as described in more detail below. The first adhesive layer has a first coat formula which is gentle on the eyelid and the second adhesive layer has a second coat formula which provides stronger adherence than the first coat formula to retain the eye patch in place over skin areas or regions which are not as sensitive as the eyelid area. The term "stronger adherence" as used herein is intended to mean that the second adhesive layer is "stickier" or has a higher tackiness than the first adhesive layer.

In an embodiment, low-level light therapy (LLLT) may be delivered to skin around the eyes to provide improved aesthetic benefit, such as, wrinkle reduction in the corners of the eyes (crow's feet) including frown and forehead lines. This provides the possibility for a triple combined therapy for MGD, LGT and wrinkle reduction.

A multi-use smart control unit (hereinafter referred to as simply "control unit") comprises at least one rigid, semi-flex or flex printed circuit board (PCB) encapsulated by a housing. At least one radiation element is connected to the PCB and configured for providing thermal (IR) and/or light energy to at least one dermal eye patch.

The control unit may comprise at least one photovoltaic cell portion for energy harvesting of ambient light. Each photovoltaic cell portion may comprise a connection with the energy source component.

The control unit may comprise one kinetic energy harvesting element for kinetic energy harvesting from walking and related body motion.

The control unit may comprise a thermo-generator harvester to recharge the energy source from body heat.

In its simplest embodiment, the control unit contains a micro universal serial bus (USB) port for direct connection via USB cable to a laptop (the laptop providing recharging, programming and data download); or to smartphone (where smartphone is used for programming and data download).

In another embodiment, the body or housing of the control unit comprises a semi-rigid or rigid plastics material. A metallic housing can also be used. The semi-rigid or rigid body or housing can readily be accepted within a pouch or cavity of a soft fabric headband. By storing the control unit within the headband, it is retained hidden in place during use. The control unit may be additionally secured by an elastic band or strip which facilitates easy and quick insertion of the control unit into the headband as well as removal therefrom.

A MCU is also mounted on the PCB and configured for controlling the operation of the at least one radiation element. The MCU may include a memory component which is configured to store data measured by a sensor component, such as, a biosensor located to determine use or wear of the system.

At least one energy source is mounted within the control unit housing and connected to the PCB and configured for supplying power to at least the radiation and/or light element and the MCU. The energy source may include non-rechargeable or rechargeable lithium coin batteries, or alternatively, rechargeable thin film micro-batteries. This has the advantage of providing a compact, low-profile self-contained device.

In another embodiment, the batteries may be non-rechargeable lithium coin batteries and the housing of the control unit is adapted to be opened for the replacement of the batteries.

In one embodiment, the battery (and/or a PCB circuit to which the battery is connected) is connected to a micro USB port accessed via an opening in the control unit housing. A standard micro USB cable can be connected to the USB port for connection to a standard power source, such as, a laptop USB port or USB connector in a wall outlet. Whilst the connection to the standard power source may be continuous for powering the system while in use, it is preferred that the connection be temporary to allow the recharging of a rechargeable battery within the control unit when the system is not being used.

In another embodiment, the USB cable can be used for data transfer between the control unit and for the programming thereof. The port may also be connected to the microcontroller component via the PCB.

In an alternative "wireless" or non-contact embodiment, an antenna or inductive coil is at least partially mounted on the PCB and connected to the microcontroller component, the antenna or inductive coil being configured for at least receiving external signals and for passing them to the microcontroller component. Signals may also be transferred out of the body or housing using the antenna or inductive coil. Power is provided to the rechargeable battery by near-field or inductive coupling through the antenna or inductive coil.

In a further embodiment, conventional capacitive coupling may be used, but will not be described here in detail. Other forms of wireless power transfer methods may also be used, such as, microwave coupling and light wave coupling.

The PCB also incorporates two switch mechanisms to activate one of several pre-programmed therapy sessions in either single or dual eye modes.

The dermal eye patches incorporate 'dual adhesion', that is, an SA skin contact layer as an inner layer for the eyelid and an outer layer for securing the periphery of the eye patch in place within the eye socket, over the eye socket or onto the face of a user depending on the size of the eye patch employed.

Each eye patch includes a multi-layer custom heating element, featuring a polyimide coated, custom 'ovalized' or circular heat element capable of sustaining up to 1W, is provided in an IR reflecting and light blocking external fabric layer. The fabric layer may be woven or non-woven and is capable of being rolled-up while having high flexibility and conformity to fit varying eye area geometries. In one embodiment, a light pipe may surround the peripheral region of the eyelid, the light pipe being connected to the control unit. Extensions to the light pipe may be provided to apply light to regions around the eye other than the eye socket. In another embodiment, additional thin light-emitting diode (LED) elements may be provided instead of or as part of the light pipe to apply extra light to regions around the eye other than the eye socket.

Whilst it is preferred that the radiation or light source(s) operate at certain wavelengths, such light components may comprise one of the following: at least one solid-state light emitting diode, at least one organic light-emitting diode; at least one quantum dot light-emitting diode. A LED may comprise two different LED emitters in one case. There are two types of these: one type where two dies are connected to the same two leads antiparallel to one another where current flow in one direction emits one color, and current flow in the opposite direction emits another color; and another type where two dies have separate leads for each die and another lead for common anode or cathode, so that they can be controlled independently.

Although wavelengths include all visible and non-visible light, the main wavelength ranges are in the red to infrared region, between 600nm and 12µm.

In one embodiment, the radiation or light source is programmable, and can be controlled to operate in accordance with a predetermined program including duration, intensity, frequency of operation, wavelength etc.

In an embodiment, the eye patch is configured to focus light, when *in-situ* on the user's eyelids and surrounding facial skin, and, to be mounted on a backing layer and rolled up and stored in the headband when not in use. An elastic band or strip may be used to retain each eye patch within the headband when not in use.

It is preferred that the eye patch comprises a soft, flexible, conforming, adherent, biocompatible material in contact with the user's skin and as a protection over the flexible heat element layer. In one embodiment, the biocompatible material comprises silicone gel adhesive.

The general shape of the eye patch can be round, oblong, oval, teardrop, etc. and may also incorporate web, slit, or perforated designs for areas where there is little need to attach (e.g. a perforation for eyebrow region).

Eye patches are designed for maximum therapeutic benefit to seal eyelid (to treat LGT) and extend underlying meibomian glands (to treat DES) while it delivers energy to underlying and neighboring vasculature (which ultimately supplies meibomian glands), and also helps melt or loosen thickened oily deposits and obstructions within the glands.

The eye patches may have different sizes and configurations - one for the eye region only and other larger versions with LLLT for aesthetic treatment of surrounding skin.

The eye patches are made in high flex layers with a final layer of silicone gel adhesive which is in contact with the eyelid skin and conforms to their shape gently for a more personalized fit every time.

The eye patches may be used individually (one eye at a time) or on both eyes simultaneously and have corresponding therapy programs under each scenario.

The eye patch converts electrical energy to thermal energy via a thin film high flex heat coil assembly within the eye patch. This thermal energy helps melt or liquefy any waxy or solid meibomian gland obstructions. The thermal energy is cyclical over the course of treatment so that when patient awakes there is limited or no crusting or re-solidification.

The thin-film flex heat coil may be configured within a polyimide, known for its excellent thermal and electrical resistance, or other substrate sandwich which may protect the skin from burns or any other adverse effects. A second insulative layer may be added over the heating layer for focusing, directing, or reflecting the heat towards the underlying skin surface.

Another adhesive layer under the heat coil assembly is provided to protect the patient from contact with the heating layer and to adhere gently to the patient's skin. These insulative layers over and under the heat coil assembly may accordingly be fabricated from a variety of insulative materials (e.g. woven and non-woven fabrics and tapes, silicone sheet and gel adhesives, polyurethane films or foams, other polymers, etc.).

Although the application of heat energy from the eye patch is described, other variations may alternatively include the application of using multi-layer eye patches for cooling of the underlying skin via endothermic reactions. For example, thermoelectric cooling uses the Peltier effect to create a heat flux between the junction of two different types of materials; a solid-state active heat pump which transfers heat from one side of the device to the other, with consumption of electrical energy, depending on the direction of the current.

Aside from delivering controlled heat or LLLT energy (or controlled cooling) from the eye patches, the eye patches may also include a layer for the diffusion or release of one or more pharmaceutical, photo-pharmaceutical, biological, photobiological, or chemical/photochemical agents (hereinafter referred to generally as "Agents") either alone or in combination with the thermal or optical radiance treatment. For instance, the Agents may be incorporated into the either the contact layer, insulative layer, or in a separate layer entirely, for transdermal delivery to the meibomian glands or to the areas surrounding the eye for additional and/or alternative treatments. In the event that the Agent is released during, for example, a heat treatment, the heat may help to improve penetration of the Agent into the underlying skin.

While the eye patches may be applied over the eyelids and their meibomian glands, variations of the eye patches may also be used to treat other skin disorders and aesthetic concerns in proximity to the eye, face, and other body areas (e.g., IR and blue light treatment patches for facial or torso acne). In such a case where no treatment is to be provided for addressing eye disorders, the control unit can simply be taped to the skin near the desired location as a wearable device without the need for the headband. Alternatively, the control unit may be located in a pouch which may be worn at a suitable position on the body of the user.

As described above, the system includes a headband in which the control unit is mounted for use. Similarly, the headband can be used to store the eye patches when not in use. The headband and eye patches are reusable and may be washable.

In an embodiment, the reusable and washable headband and eye patch components have their outer surfaces treated with an anti-microbial coating.

In order to reflect and direct thermal or light radiance more efficiently, surface areas of the eye patches include at least one surface coated with a reflector mirror film while the outside of eye patch can be made of a dark or black cloth to prevent any unwanted light to eyes and to reflect thermal or IR heat back toward eye (using IR reflective fabric) as an outer layer.

The therapy system provides many advantages over the prior art solutions as will become apparent from the description below.

In treating conditions such as meibomian gland dysfunction or dry eye syndrome, an eye patch can be gently adhered to the skin of the upper and lower eyelids and surrounding structures to deliver heat or other forms of energy, moisture retention, etc. (alone or in combination) to the one or more meibomian glands contained within the underlying skin. In particular, the assembly for the treatment comprises headband, control unit, and one or two, multilayer, high flex, eye patches.

It will be readily appreciated that other glands contributing to tear film production (e.g. the lacrimal gland and/or palpebral lacrimal gland located above the eye) may also benefit from the eye patches.

The control unit may also be configured to incorporate an alert when the therapy session has completed, e.g., LED light pipe blink, audible alarm or vibration from the control unit.

In heating conditions such as meibomian gland dysfunction (MGD), which is commonly associated with the evaporative form of dry eye syndrome (DES), a patch device can be affixed to the skin of the upper and/or lower eyelids to deliver thermal energy, LLLT, cooling or other forms of energy, and various forms of agents, etc. (alone or in combination) to the one or more glands and wrinkles contained within the underlying skin.

The device is primarily designed for dual eye, cyclical session, night time therapy during sleep. However, single sessions during daytime are possible in either dual or single eye mode. In single eye mode, the user can alternate therapy so that the non-patched eye can blink and provide physiological benefits of clearing the freshly liquidated meibum through natural biomechanical action. At the same time, one eye treatment allows a user to continue with daily activities.

These sessions may be set by user via a switch for 5-10-15 minutes of thermal energy therapy (to one or both eyes) depending on need as some users may have more difficult meibum flow obstructions than others and require more therapy time. Users can utilize the reusable and washable patches for up to 20 wear sessions and then dispose of the eye patches and replace with fresh ones as needed. Naturally, each eye patch can be disconnected from the control unit when it needs to be cleaned or replaced. In this way, particularly when cleaning, there is no risk of water damaging the control unit when the eye patches are cleaned. The rechargeable, re-usable, smart control unit and headband allows for therapy anywhere and anytime and for use as many times as needed to provide improved outcomes and better eye health.

Delivery of thermal IR and LLLT (red light) energy has been shown to promote vasodilation, increase Adenosine Triphosphate (ATP) synthesis in cell mitochondria to providing more energy for cellular activity, improve blood flow, reduce chronic inflammation and improve overall tissue health. This also bodes well for user suffering from chronic inflammation of ocular surface, conjunctiva, cornea, or deeper eye conditions where reducing inflammation and neovascularization is important such as wet Age-related Macular Degeneration (AMD) and Diabetic Retinopathy (DR). Whereas silicone gel sheets have been shown to improve hydration and apply light compression to reduce fine lines and wrinkles in skin while also improving scar appearance particularly scars which are hypertrophic.

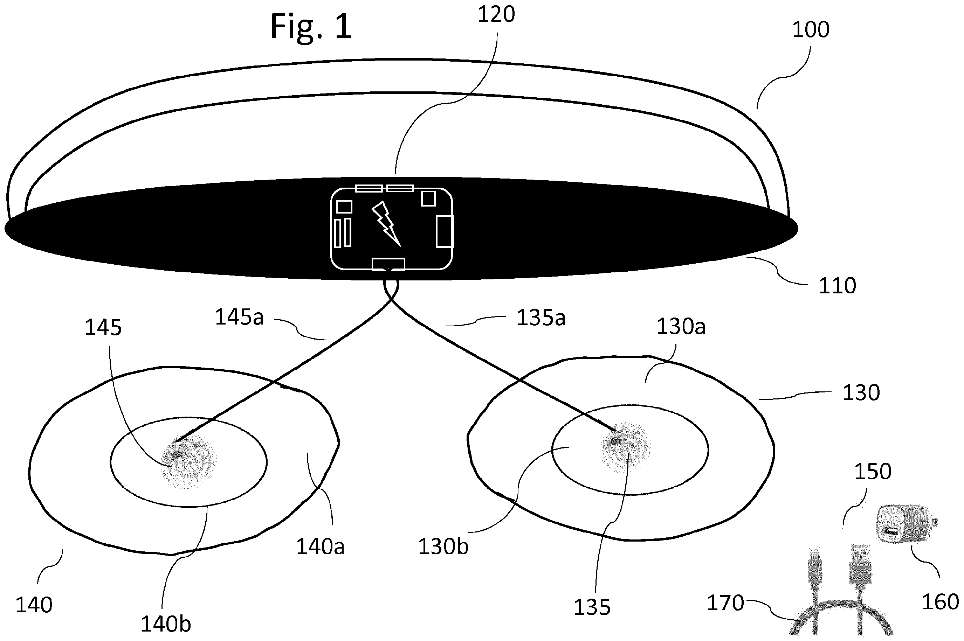

Referring to the drawings, Fig. 1 is a schematic illustration of a therapy system 100. The system 100 comprises a headband 110 configured to house a control unit 120 which is connectable to eye patches 130, 140 by way of respective electrical connections 135a, 145a. Each electrical connection 135a, 145a may be housed in a waterproof lumen so that it is not damaged during washing of the eye patch 130, 140. For example, the waterproof lumen in this case may be a plastic-insulated copper wire where plastic insulation forms the lumen and the copper wire forms the electrical connection. Examples of plastic materials which may form the plastic insulation include polyvinyl chloride (PVC) and polyamides, such as nylon.

Each eye patch 130, 140 comprises an outer area 130a, 140a, which is configured to adhere to an area surrounding an eyelid of a user, and, an inner area 130b, 140b, which is configured to adhere to the eyelid of a user. The inner area 130b, 140b can be considered to be the eyelid area and includes an element 135, 145 through which thermal (IR) or visible light is applied to the eye on which the eye patch is adhered. Each element 135, 145 is connected to the control unit 120 via the electrical connections 135a, 135b as described above.

As described above, the outer area 130a, 140a and the inner area 130b, 140b of each eye patch 130, 140 forms a dual adhesive system where the inner area only has an adhesive for locating and gently adhering the eye patch on the eyelid and the outer area has an adhesive for adhering the eye patch to the area surrounding an eyelid of a user, for example, the skin in the eye socket. As described below, the outer area may extend over a larger area than the eye socket.

The control unit 120 is configured to be recharged by connection to a USB charging system 150, the USB charging system comprising a charger 160 and a charger cable 170 as shown.

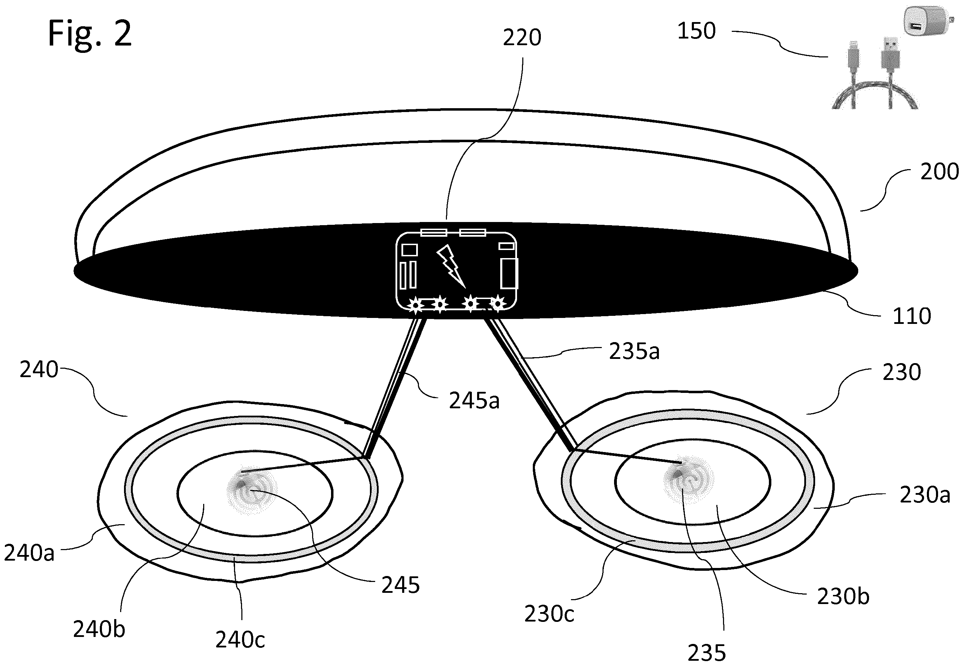

Fig. 2 illustrates a system 200 which is similar to Fig. 1 but having a different eye patch configuration. Components described previously with respect to Fig. 1 are referenced the same.

The system 200 comprises a headband 110 and a charging system 150 as described above with reference to Fig. 1, and a control unit 220 which is connectable to eye patches 230, 240 by means of a dual purpose connection 235a, 245a. Each eye patch 230, 240 is similar to respective ones of eye patches 130, 140, and, each has an outer area 230a, 240a and an inner area 230b, 240b with an element 235, 245 located in the inner area 230b, 240b and connected to the control unit 220 by way of the dual purpose connection 235a, 245a.

In this embodiment, the eye patches 230, 240 include a light pipe 230c, 240c located in the outer area 230a, 240a and which surrounds the eye. The light pipe 230c, 240c is configured as a loop connected to the dual purpose connection 235a, 245a. Here, light generated by the control unit 220 is transmitted along dual purpose connection 235a, 245a to respective ones of the light pipes 230c, 240c.

Each dual purpose connection 235a, 245a may comprise a two-channel lumen where one channel carries an electrical connection from the control unit 220 to an associated element 235, 245 in the eye patch 230, 240, and, the other channel forms a light pipe through which light is directed to the light pipe 230c, 240c in the connected eye patch 230, 240. For example, the two-channel lumen may be formed by an extruded tubing having two or more internal channels, and, a plastic-insulated copper wire, forming the electrical connection, is threaded to fill or fit through one internal channel or lumen while one of the other internal channels or lumens remains open and forms the light pipe.

Although a two-channel lumen is described, the lumen may be a multi-channel lumen with each type of radiation being arranged to pass along an individual separate channel within the lumen.

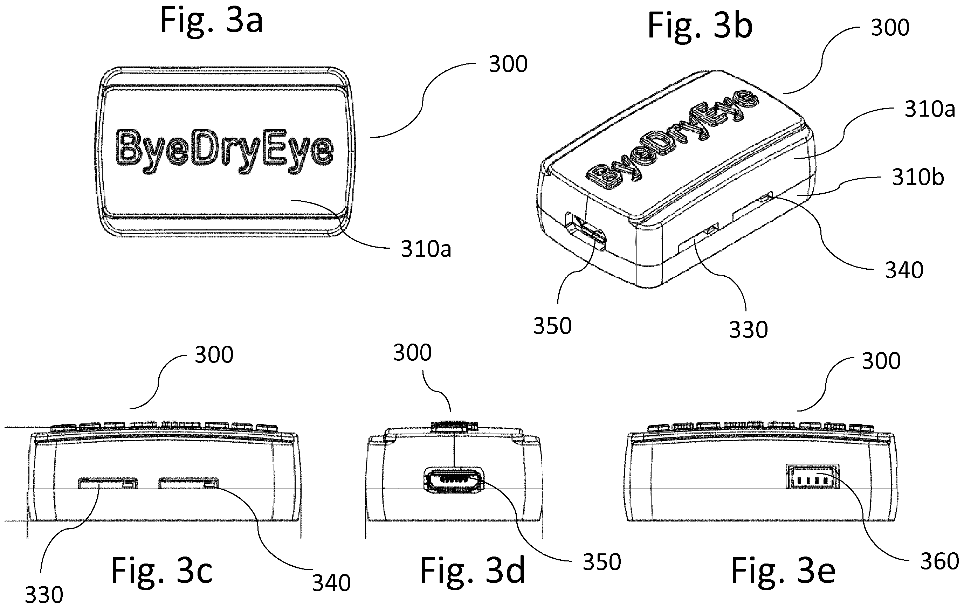

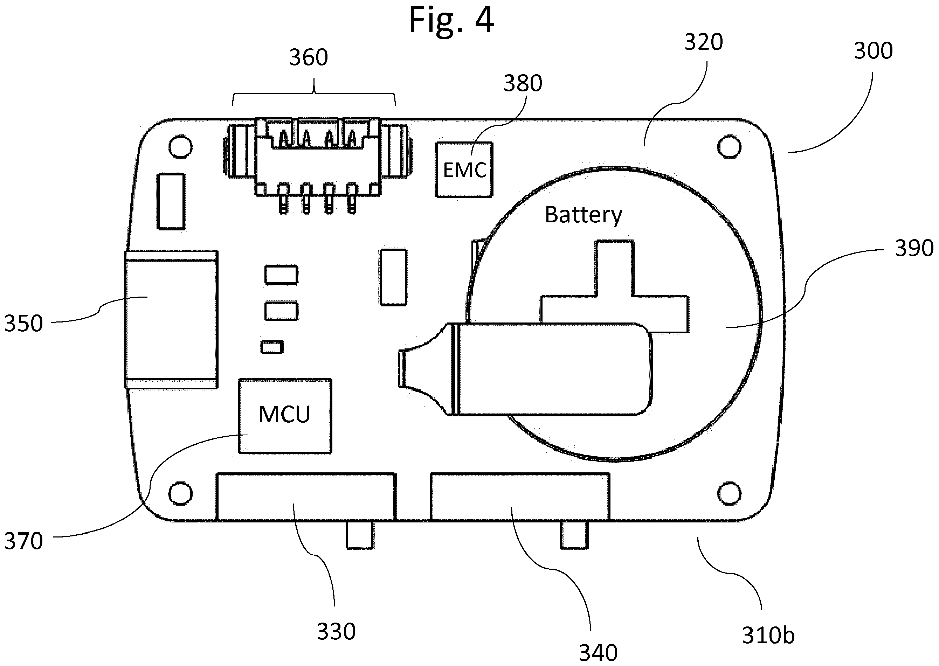

A proprietary control unit 300 is shown in Figures 3a to 3e. The control unit 300 comprises a housing 310 in which a PCB 320 (shown in Fig. 4) is mounted. The housing 310 comprises an upper portion 310a and a lower portion 310b. The upper and lower portions may be separable for the replacement of a battery if it is not rechargeable as discussed above.

Such a control unit 300 comprises a two-piece housing as described below having approximate dimensions of 40mm long by 26mm wide and 14mm high.

The PCB 320 is connected to switches 330, 340, micro USB port 350 and eye patch connectors 360. The capacitive touch sensors and/or temperature sensors may be mounted on or connected to the PCB 320 of the control unit 300 which is preferably mounted within the headband 110 (described above with reference to Figures 1 and 2). A more detailed view of the inside of the housing 310 is shown in Fig. 4 where components described in Figs. 3a to 3e are referenced the same.

In Fig. 4, the lower portion 310b of the housing 310 of the control unit 300 is shown. The control unit 300 includes a microprocessor 370, an energy management integrated circuit (EMC) 380 and a battery 390 (rechargeable or disposable) mounted on the PCB 320. Other components such as resistors, capacitive touch sensor electrodes and/or temperature sensors, hardware and software/firmware are not shown in detail.

It will readily be appreciated that not all the electrical/electronic components supporting the operation of the control unit 300 are shown for clarity nor are they described in detail for ease of explanation.

Switches 330, 340 are therapy setting switches and provide control signals for the microprocessor 370 so that an appropriate therapy can be provided in accordance with those control signals.

The micro USB port 350 is configured to receive a micro USB connector of the charging cable 170 of the charging system 150 for charging a rechargeable battery 390, and, a micro USB connector connected to an external processor (not shown) for the transfer of data between the microprocessor 370 and the external processor.

Light pipe connectors are provided in the eye patch connectors 360 which provide LED light pipe coupling as well as electrical coupling for the eye patches (shown in Figs. 1 and 2). As described above, the light pipe connectors may comprise multi-channel lumens configured to provide a separate path for the electrical connections and one or more light sources.

The capacitive touch sensor electrodes and/or temperature sensors (not shown) are used to monitor parameters of the user and for providing information relating to those parameters to the microcontroller 370. As described above, the microcontroller 370 preferably includes a memory for storing such information prior to its transfer to the external processor as described above.

The software/firmware is programmed for controlling power applied to the heat element (approximately 275mW per eyepatch) to a safe and approximate 45°C temperature to allow a therapy session to achieve an approximate 8°C temperature increase of the underlying skin, and to control the LLLT sessions to up to 1J/cm² of red light therapy for up to 30 minutes as a 'one-time' treatment session or shorter cyclical treatment sessions delivered over a longer multi-hour period of time as needed during an overnight sleep session.

The control unit features manual switches 330, 340 to allow a user to quickly and easily set therapy sessions for single or dual eye treatment including treatment length.

Alternatively (or additionally), the control unit 300 may include a RF wireless (Bluetooth) capability to program desired therapy type and record therapy session data captured by sensors into memory chips (not shown) on the PCB 320 prior to being transferred to an external processor, such as, a handheld smart phone or other device periodically or on demand, with the aid of a corresponding software application developed for the device. Moreover, the control unit 300 may also have biosensors (not shown) extending from its housing 310 by wires (or alternatively linked wirelessly) which can be strategically placed along the headband, for example, above the temples for measuring heart rate of the user.

The control unit 300 also contains indicators (such as LEDs) programmed to indicate charging status, end of therapy, battery condition, etc. These components are not shown in the drawings.

An antenna element (also not shown) may be provided in the control unit 300 for receiving programming from an external source. Information stored in a memory of the MCU may also be transmitted from the control unit 300 to such an external source using the antenna element. In addition, the antennal element may be used for inductive coupling with an external element to provide power to the battery 390 under the control of the EMC 380.

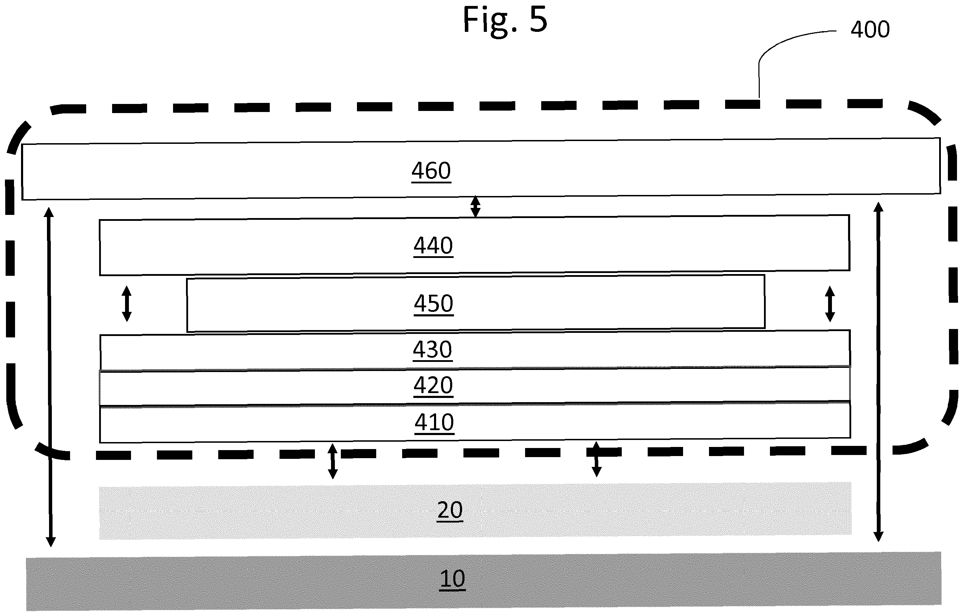

Fig. 5 illustrates a section through an eye patch 400 in accordance with the present invention. The eye patch 400 comprises a plurality of layers which are configured for being placed over and adhered to a region 10 of a user including a closed eyelid 20 of the user. The eye patch 400 comprises a silicone adhesive (SA) layer 410 for adhering to the closed eyelid 20. The SA layer 410 has a polyurethane (PU) backing layer 420. A permanent adhesive layer 430 mounts the backing layer 420 to an IR reflecting and light occluding dark woven layer 440 sandwiching a flexible IR coil element substrate 450 (which can be energized) therebetween. The woven layer 440 is adhered to a silicone adhesive SA layer 460 which adheres the eye patch 400 to non-eyelid skin and/or hair, for example, the region 10 (which may be the skin within an eye socket of the eye being treated).

The flexible IR coil element substrate 450 may comprise a printed tracer element sandwiched in a layer of polyimide. Alternatively, the tracer element may be sandwiched in silicone or mica or any multi-layer combination thereof. A thin coating of graphene, typically only a few nanometers thick, may be added to or incorporated into the substrate 450.

The SA layer 410 comprises a `low coat' formula which is not as adhering as the SA layer 460 which comprises a 'high coat' formula, and, as such, form a dual adhesive system which is gentle on the eyelid for location of the patch and more robust in the region surrounding the eyelid, for example, skin within the eye socket (or other area to which the patch is adhered) to ensure that the eye patch remains in place until removed. The 'high coat' formula has a stronger adherence than the `low coat' formula, that is, the 'high coat' formula is stickier or tackier than the `low coat' formula.

By having such a dual coat system on the eye patch, maximum comfort (especially during removal), adherence, reusability, and durability can be obtained.

An additional graphene nano-layer (not shown) may be incorporated into the coil element substrate 450 (e.g. as a thin 100nm or less coating on the polyimide layer or multi-layer as described above) or as a coating on another layer (e.g. the dark woven layer 440 or the permanent adhesive layer 430) to increase thermal conductivity and to make more efficient and more uniform heat transfer to the underlying treatment area (e.g. eyelids and surrounding tissue).

As electronic devices become increasingly miniaturized, thermal management becomes more critical. Efficient heat dissipation guarantees optimal performance and service life of these miniaturized devices. Graphene nanoplatelets (GnPs) have excellent thermal properties and can be used in the fabrication of polymer nanocomposites where high thermal conductivity is required. Due to its unconventional electron confinement and high mobility, graphene, an atomically thin, single-layer carbon sheet, also possesses unique optoelectronic properties.

A diamond patterned, reusable release sheet or layer (also not shown) may be provided to which the eye patch can be adhered when not in use. This enables the eye patch to be kept clean, and can be folded or rolled up for storage when not in use, that is, when not on the eye. The diamond patterned release sheet or layer may be made from low density polyethylene (LDPE), polyester (PET), high density polyethylene (HDPE), polypropylene (PP) and polyvinyl chloride (PVC).

A light pipe (not shown) may also be provided in the eye patch 400 between the permanent adhesive layer 430 and the dark woven layer 440 and is located in the outer area of the eye patch as described above with reference to Fig. 2.

The light pipe is intended to provide red light therapy to address aesthetic issues in the eye area, for example, crow's feet at the corner of the eyes, frown lines on the forehead (a larger eye patch is required as described below), and eye bags. The light pipe may comprise a silicone ribbon located in the outer area of the eye patch as described above.

The eye patch 400 may be fabricated from different materials including silicone gel layers of different coat densities and adhesive strength to better match skin elasticity and removal comfort, polyurethane, woven/nonwoven interior reflective layer (for IR and visible light reflection to skin surface, biocompatible adhesives, polyimide, polyethylene, silicone, graphene, black woven/non-woven exterior layer to block out unwanted ambient light, etc. These layers also serve to insulate, distribute heat more uniformly, and provide protection from burn or discomfort on either side of the heat coil element.

Alternatively, the eye patches may be used with any number of agents applied to the skin for various combination therapies. The skin contact layer of the eye patches may also be impregnated with one or more agents for transdermal release, or non-leeching agents such as an anti-microbial coating.

The flexible, resistive, heat coil element, typically having an approximate diameter of 20mm, is configured to cover the eyelid area and convert electric power supplied from the rechargeable battery in the control unit into a temperature increase to between 40 and 50°C, preferably between 40 and 45°C, that is, an approximate 6 to 8°C increase in normal eyelid skin temperature of between 36 to 37°C within two minutes. The control unit may supply up to 1W of electrical power in total to supply each heating element with up to 500mW of electrical power when operating in a dual eye mode. Typically, the control unit supplies 550mW of electrical power with approximately 275mW for each eye patch when in the dual eye mode so that the eyelid temperature may reach around 42°C as a minimum.

Optionally, various sensors for temperature, humidity, pressure, could be incorporated into the eye patch layers to provide additional feedback to the control unit, but, as the eye patches are disposable after a number of uses, this would add to the overall cost of each eye patch.

Alternatively, a cooling element (via Peltier effect) may be applied for conditions that benefit from vasoconstriction to reduce blood flow or leakage.

Therapeutic agents which may be incorporated into the eye patch layers may include, but are not limited to, anti-inflammatory compounds, antibiotics, tetracycline, corticosteroids, enzymes, peptides, vitamins, oils, lipids, retinol, acids, proteins, minerals, etc. whether or not they are activated or aided photonically (by light) and/or thermally (by heat/IR).

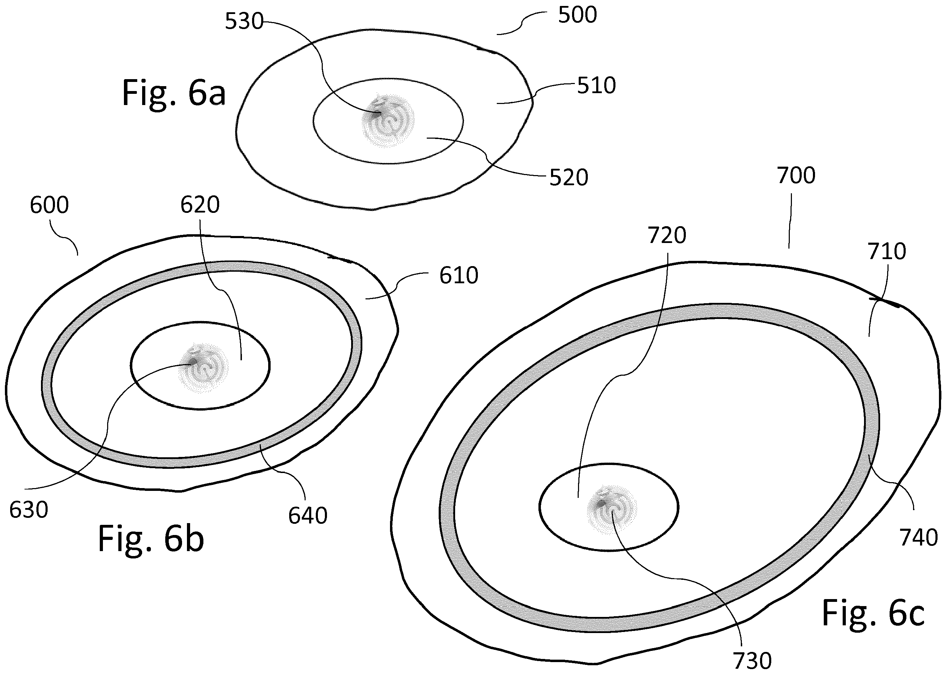

Figs. 6a to 6c are schematic illustrations of different eye patch configurations. The connections between the eye patches 500, 600, 700 and the control unit are not shown for clarity.

Fig. 6a illustrates a small eye patch 500 configured to fit over a closed eyelid within the eye socket. Eye patch 500 can be used for DES/MGD/LGT therapy. The eye patch 500 comprises an outer area 510, an inner area 520 and an element 530 located in the inner area 520. As described above, the element 530 is a heating element connected to the control unit by way of an electrical connection as described with reference to Figs. 1 and 2 above. A typical size for the eye patch 500 is approximately 50mm wide and 50mm long for the eyelid and meibomian gland heating. As described above, the inner area 520 is intended to be adhered to the eyelid of a user with the outer area 510 being adhered to skin surrounding the eyelid, for example, skin in the eye socket area.

Eye patch 600 is a larger (or mid-size) eye patch which is intended to extend around and over the eye socket and not fit on the skin surrounding the eyelid within the eye socket like eye patch 500. Eye patch 600, apart from providing DES/MGT/LGT therapy, also provides LLLT to treat crow's feet, eye bags and frown lines. The eye patch 600 comprises an outer area 610, an inner area 620 and an element 630 located in the inner area 620. As described above, the element 630 is a heating element connected to the control unit by way of a dual purpose connection as described with reference to Fig. 2 above. In addition, a light pipe 640 is provided in the outer area 610 for directing light to the area around the eye of the user to provide red light therapy. As described with reference to Fig. 2, the light pipe 640 is also connected to the control unit by the dual purpose connection. A typical size for the eye patch 600 is approximately 75mm wide and 75mm long and adds more coverage for the lacrimal (orbital and palpebral) glands as well as adding LLLT for wrinkle therapy. As described above, the inner area 620 is intended to be adhered to the eyelid of a user with the outer area 610 extending outwards from the eyelid and adhering to surrounding skin (or hair) for LLLT.

Eye patch 700 is a large (larger than eye patch 600) eye patch which is intended to extend up to the forehead and to adhere to skin surrounding the eyelid of a user, for example, skin within the eye socket like eye patch 500. Eye patch 700, apart from providing DES/MGT/LGT therapy and treating crow's feet, eye bags and frown lines, also provides LLLT for treating tear troughs and forehead lines. The eye patch 700 comprises an outer area 710, an inner area 720 and an element 730 located in the inner area 720. As described above, the element 730 is a heating element connected to the control unit by way of a dual purpose connection as described with reference to Fig. 2 above. In addition, a light pipe 740 is provided in the outer area 710 for directing light to areas around the eye of the user to provide red light therapy. As described with reference to Fig. 2, the light pipe 740 is also connected to the control unit by the dual purpose connection. A typical size of eye patch 700 is approximately 100mm wide and 100mm long and further adds LLLT for wrinkle treatment up to the forehead lines. As described above, the inner area 720 is intended to be adhered to the eyelid of a user with the outer area 710 extending outwards from the eyelid and adhering to surrounding skin (or hair) for more extensive LLLT.

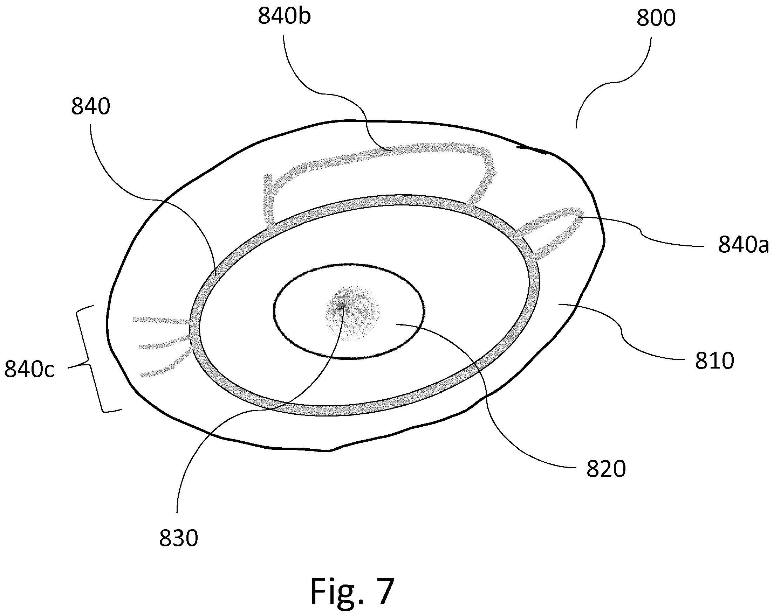

Fig. 7 illustrates an eye patch 800 which is similar to Fig. 6c but includes additional light pipes. As before, the connections to the control unit are not shown for clarity.

Eye patch 800 is intended to extend up to the forehead and not to only be adhered to skin surrounding the eyelid, for example, within the eye socket like eye patch 500. The eye patch 800 comprises an outer area 810, an inner area 820, an element 830 located in the inner area 820, and, a light pipe 840 provided in the outer area 810 for directing light to areas around the eye of the user to provide red light therapy. As described above, the element 830 is a heating element connected to the control unit by way of a dual purpose connection as described with reference to Fig. 2 above. In addition, the light pipe 840 includes branches 840a, 840b, 840c which are intended to target specific wrinkle areas for red light therapy or LLLT. For example, branch 840a may be used to target frown lines, branch 840b to target forehead lines, and, branch 840c to target crow's feet. As described with reference to Fig. 2, the light pipe 840 is also connected to the control unit by the dual purpose connection and provides light to each of the branches 840a, 840b, 840c. As described above, the inner area 820 is intended to be adhered to the eyelid of a user with the outer area 810 extending outwards from the eyelid and adhering to surrounding skin (or hair) for LLLT so that the light pipe branches 840a, 840b, 840c are positioned on the skin at the areas to be treated.

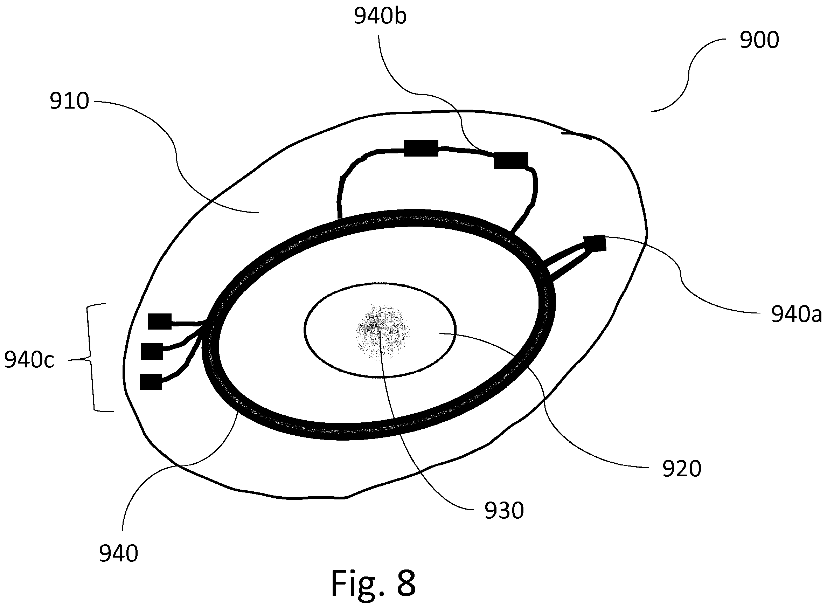

Fig. 8 illustrates an eye patch 900 which is similar to Fig. 7 but includes additional LEDs within the eye patch itself. As before, the connections to the control unit are not shown for clarity.

Eye patch 900 is intended to extend up to the forehead and to adhere to skin surrounding the eyelid of a user, for example, skin within the eye socket like eye patch 500. The eye patch 900 comprises an outer area 910, an inner area 920, an element 930 located in the inner area 920, and, a light pipe 940 provided in the outer area 910 for directing light to areas around the eye of the user to provide red light therapy. As described above, the element 930 is a heating element connected to the control unit by way of a dual purpose connection as described with reference to Fig. 2 above. In addition, the light pipe 940 includes branches 940a, 940b, 940c which are intended to target specific wrinkle areas for red light therapy or LLLT. In this embodiment, additional ultra-thin LEDs are provided in each of the branches 940a, 940b, 940c. As described with reference to Fig. 2, the light pipe 940 is also connected to the control unit by the dual purpose connection. As described above, the inner area 920 is intended to be adhered to the eyelid of a user with the outer area 910 extending outwards from the eyelid and adhering to surrounding skin (or hair) for LLLT so that the light pipe branches 940a, 940b, 940c and their associated ultra-thin LEDs can be positioned on the skin at the areas to be treated.

Although eye patch 900 has been described as having a light pipe/localized LED combination, a variant thereof may comprise the ultra-thin LEDs only with electrical connections being provided from the control unit by way of the dual connection as described above. It will readily be appreciated that any combination of light pipe and/or LEDs may be used together with the heating element which addresses DES/MGT/LGT.

Such ultra-thin LEDs may have a thickness of less than 0.5mm and may be designed using printed conductive ink and are connected to the control unit by way of thin wires. These LEDs may also be placed and connected by tracers printed on an extended version of the flexible, thin substrate (e.g. polyimide) of the flexible energized IR coil element substrate 450 as shown in Fig. 5.

Generally, irrespective of the size of the eye patch, each eye patch is flexible, adherent to the skin surface of a user and conforms to facial contours etc.

Eye patches may be used to potentially treat eye disorders beyond DES/MGD/CK/LGT such as blepharitis, conjunctivitis, iritis, retinitis, uveitis, and for post eye or eyelid surgical procedures where combination or individual LLLT and thermal energy with hydration therapy may benefit in accelerating healing and improving outcomes.

Eye patches may also provide programmed pulsating energy delivery to the meibomian glands to generate micro-mechanical pressure to facilitate meibum release and clearing any obstructions. Besides electromagnetic energy in the visible and infrared wavelengths, micromechanical elements can be incorporated behind the skin contact layer of the eye patch to generate vibrational energy or micro-movement from micro-actuators, biphasic materials, shape memory materials, transducers etc. This vibrational energy is converted from the electrical energy in the rechargeable battery within the control unit, and it may be in addition to, or separate from, the thermal and LLLT energy treatment. Other forms of energy can also be incorporated into the eye patches including ultrasonic, RF, microwave, magnetic, etc.

The high flex heat coil of approximately 20mm diameter is thin and flexible to conform with the other layers of the eye patch including the adhesive layer that contacts the eyelid tissue and provides heat therapy to the meibomian glands. The heat coil can be further customized in varying geometries to widen the treatment area (e.g. adding direct coverage of the lacrimal glands). The heat coil is directly connected to the control unit worn in the headband and powered by a rechargeable battery.Whilst the eye patches of the present invention as described above with reference to Figs. 5 to 8 may be configured and sized specifically for intended area of coverage in the form of a tapered 'ovalized' shape, the shape of the eye patches is not so limited to other non-symmetric shapes may be utilized in order to maximize coverage area.

The application of thermal energy (radiant and conductive) passes through vascularized and moist eyelid tissue quite effectively to the glands. This low level energy may also have beneficial effects for other ocular surface disorders or improve post-operative healing of tissue after eye or eyelid surgery.

The thickness of the high flex, thin, roll-able, multi-layer, eye patch is approximately 1mm, yet may be up to 3mm, and the eye patch rests against skin in a conforming manner thus removing any protrusions or potential obstructions. This thickness is less than typical warm compresses, goggles, and sleep masks - especially energized sleep masks. As the eye patches conform to anatomical curvature of skin surface, the width and length dimensions given above are larger than straight line measurements of human facial regions.The eye patches may be used by a user without the need of any assistance from doctor or caregiver.

The eye patches may have a single male connector coupled to the female power port of the control unit for providing power to both eyes. Alternatively, each eye patch may have its own connector especially with the LED coupling LLLT versions.

In LED coupling LLLT versions of the eye patch, a female connector component on the control unit allows for the insertion and 'coupling' of the light pipe connector (with limited air space to maximize radiant flux capture) to a male receptacle on the light pipe loop at perimeter of eye patch. It will be appreciated that the connectors may be the other way around provided they are complementary. As described above, the light pipe connector may be a multi-channel lumen which is configured to extend between the control unit and each eye patch. For example, the light pipe connector may form one channel of the multi-channel lumen and the electrical connection may be located in another channel of the multi-channel lumen. The channel carrying the electrical connection may comprise a plastic-coated wire joined to one or more plastic tubes for light pipe connections, or may be a plastic-insulated wire located within a plastic tube forming the channel.

The light pipe is constructed to allow the diffusion of radiation or light therethrough with additional structured layers or patterns for further enhancing uniformity in the radiation or light diffusion. Such structure layers may include cladding, gratings and/or reflective layers. The light pipe may also be "branched" from the central conduit to better distribute the light to the specific locations of the wrinkles as described above.

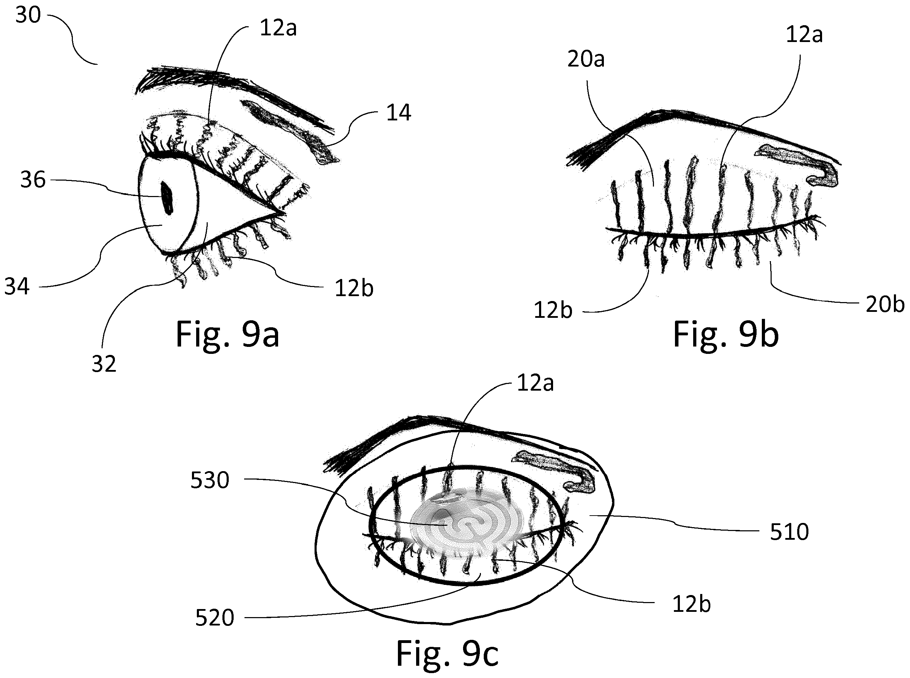

Fig. 9a is a schematic illustration of an eye 30. The eye 30 includes a sclera 32, cornea 34, pupil 36 and upper and lower eyelids 20a, 20b (as labelled only in Fig. 9b for reasons of clarity). The locations of the meibomian glands 12a in upper eyelid and 12b in lower eyelid and the lacrimal glands 14 are also shown with respect to the eye 30.

Fig. 9b illustrates a closed eye 30 where the upper and lower eyelids 20a, 20b are shown together with the associated meibomian glands 12a, 12b. As described above, the upper meibomian glands 12a comprise about 31 glands on average, are about twice the length (typically, around 5.5mm), and represent twice the volume (typically, around 26µl) compared to the lower meibomian glands 12b which comprise about 26 glands on average, are 2mm in length, and 13µl in total volume.

Fig. 9c illustrates the eye patch 500 of Fig. 6a located over a closed eye 30. As shown, the element 530 sits over the closed eyelids and meibomian glands 12a and 12b, and the adhesive in inner area 520 helps to locate the eye patch correctly before the adhesive in the outer area 510 is pressed firmly in place on skin surrounding the eyelid in the eye socket 10 as described with reference to Fig. 5 above.

As shown in Fig. 9c, the meibomian glands 12a in the upper eyelid and the meibomian glands 12b in the lower eyelid are at their most extended when the eyelids are fully closed. A partially closed eyelid would lead to eyelid skin folds and compressed or folded meibomian glands.

The eye patches may be universal, that is, they can be used for either the left eye or the right eye. Alternatively, the eye patches can be handed, that is, a left eye patch is used for the left eye and a right eye patch is used for the right eye.

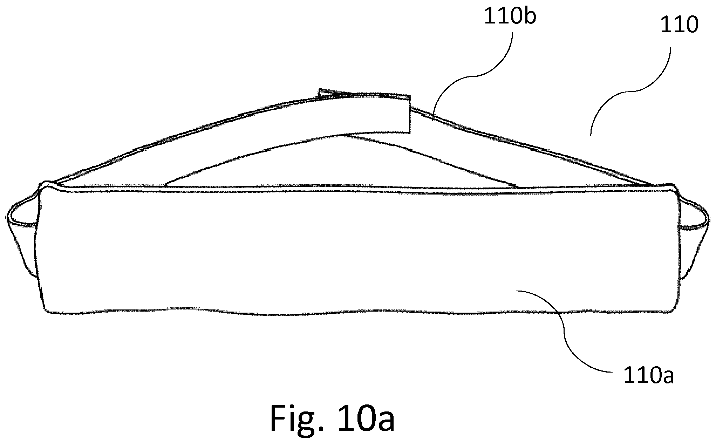

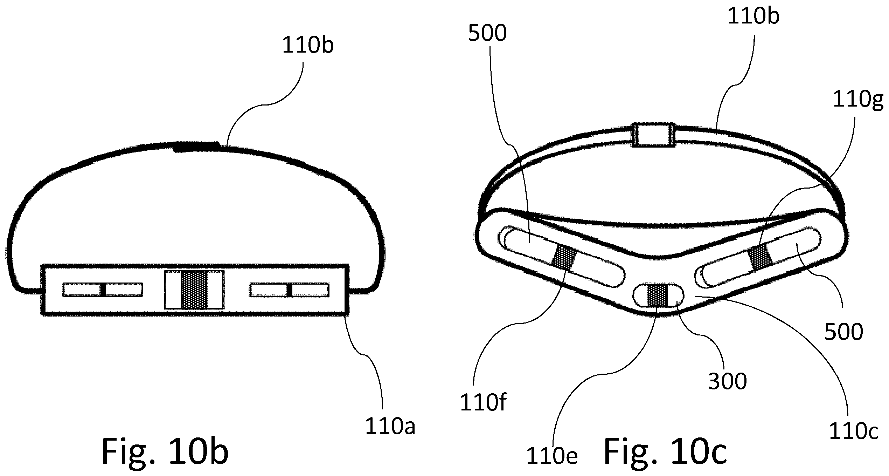

Figs. 10a to 10c illustrate views of a headband 110 as used in the systems of Figs. 1 and 2. The headband 110 comprises a front portion 110a and an adjustable rear portion 110b. The front portion 110a includes a cavity 110c having a closable opening (not shown). Control unit 300 can be located together with any biosensors (not shown) within holders located inside the cavity 110c. The cavity 110c can also be used to store eye patches when not in use making the system easy to transport and store.

Preferably, the opening is closed using one or more hook-loop fasteners or button snaps. This provides a soft edge to the headband 110 for comfort during prolonged use, for example, during a sleep cycle.

The cavity (or pouch section) 110c of the headband 110 may be up to 32cm in length (to cover temple to temple) and 4cm in width. The rear portion 110b of the headband may comprise a stretch band section configured to extend approximately 28cm across the back of the head of a user.

As described above, the cavity or pouch section 110c holds and hides the removable control unit, and one or both, roll-able or foldable, eye patches. A stretch band retainer 110e sewn into the cavity or pouch section 110c of the headband 110 helps secure the control unit 300 in place within the headband.

Similarly, a pair of stretch band retainers 110f, 110g are provided on either side of the stretch band retainer 110e for storing the rolled up eye patches when not in use. Although not shown, the cavity or pouch section 110c may also contain biosensors or electrodes along its length (e.g. above the temple) which extend from the control unit 300 and attach into button-snaps sewn into the headband 110.

The front portion 110a of the headband may be made from various fabrics, which allow a light stretch, soft interface, and periodic washing. This fabric may incorporate a foam liner (not shown) for added cushion and comfort. As described above, the rear portion 110b of the headband may comprise a pair of thin fabric stretch bands attached to the front portion 110a, and may incorporate a loop-hook closure system (e.g. Velcro^{®}) to secure the headband 110 gently onto a wide range of human head circumferences, for example, between 52cm and 59cm range which covers the vast majority of adult heads.

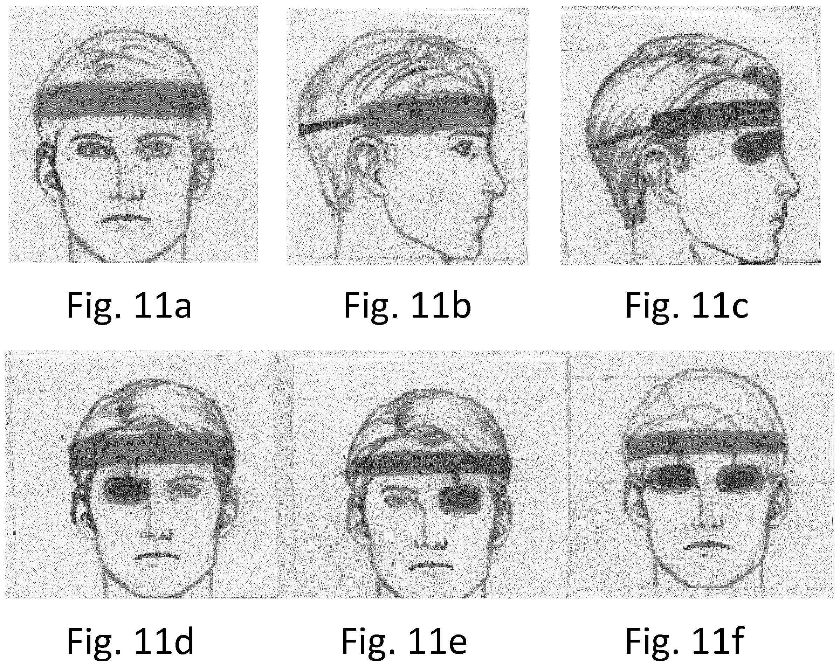

Figs. 11a to 11f are schematic illustrations of a user wearing the headband 110. In Figs. 11a and 11b, a front and side view of a user is shown. In Fig. 11c, a side view of a user wearing at least one eye patch is shown. In Figs. 11d to 11f, front views of a user using an eye patch over the right eye, over the left eye and over both eyes are shown.

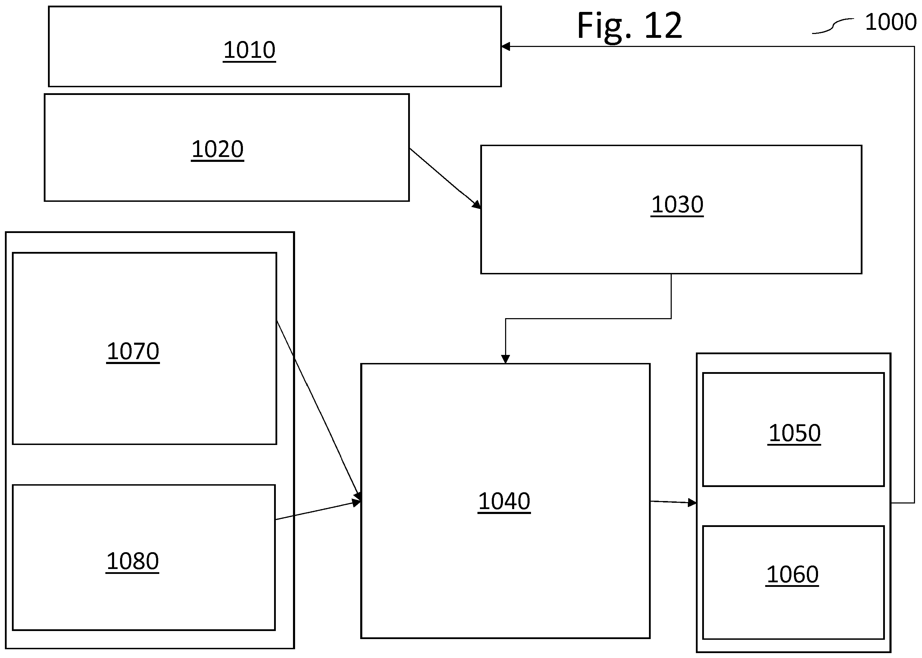

Fig. 12 illustrates a flow chart 1000 illustrating steps in a non-claimed method using the therapy system of the present invention. In step 1010, for first time operation, the control unit 300 is charged using the USB charger 150 as shown in Figs. 1 and 2. The micro USB end of the charging cable 170 is plugged into the micro USB port 350 in the control unit 300.

Once the control unit 300 is fully charged, it is disconnected from the USB charger 150, placed in the headband 110 and connected to the eye patches, step 1020. Also in step 1020, a therapy mode is selected by a user using the switches 330, 340 in the control unit 300 to activate a treatment session. In step 1030, the user removes the eye patches from their release films or layers, places the headband on his head, and places the eye patches gently onto the eyelids and surrounding skin. The therapy session is then initiated as a pre-programmed energized (IR heat and red light) and compressive hydration therapy to the eyelid tissue and surrounding tissue (depending on the size of the eye patch as described above) in step 1040. After the therapy session is over (a single therapy cycle or repetitive therapy cycles), the eye patches are removed from the eye area and placed back onto the release layer in step 1050. The eye patches are rolled up and placed inside the headband 110 for storage until next use, step 1060, and occasionally detached from control unit and washed with liquid soap and water, dried and re-attached to control unit, or alternatively discarded and replaced as necessary. The headband is removed and the rolled-up eye patches are inserted into the cavity or pouch section 110c for storage until next use. If the control unit needs to be recharged, step 1010 is carried out. Otherwise, steps 1020 to 1060 and, optionally steps 1070 and 1080, are repeated as required.

During the therapy session in step 1040, parameters are determined to assess user adherence to the therapy in step 1070. Measurements are taken from capacitive touch sensors and/or temperature sensors located in the headband 110 and stored. Measurements relating to heart rate at the temple of the user can also be measured and stored using a photoplethysmography (PPG) IR sensor within the headband 110 (step 1080).

It will readily be understood that although the method has been described with reference to using two eye patches, a similar method applies for the use of a single eye patch.

As described above, the therapy system is not limited to the treatment of DES/MGD/LGT and can, in addition, be used for aesthetic purposes. Aesthetic improvement of and around the eyelids is of high interest to both men and women especially as they age. The 2016 annual report from the American Society for Aesthetic Plastic Surgery listed eyelid surgery as the #1 procedure for those 65 and older (coincidentally an age where DES and MGD is most common). It is the 3^{rd} most common procedure for men and the 5^{th} most common for women. It is the 4^{th} most common procedure overall, and more common than nose surgery or facelifts in both sexes.

For crow's feet, frown and forehead line wrinkle reduction (common wrinkles around the eye) there are numerous products available today - many of which are hydrating silicone sheets like SilcSkin ^{®}. There are also many LED LLLT devices and face masks (in-clinic and home use) to do same.

Eye patches that combine the benefits of skin adhered, hydrating, silicone gel sheets with LLLT (red light therapy) into one comfortable wearable combination device for home-use, and uniquely designed for the skin over and surrounding the eyes, is of interest - especially if they simultaneously treat other common disorders such as DES, MGD, and LGT - especially in an aging population where prevalence of these conditions and disorders are high.

## Claims

1. An eye patch (400; 500; 600; 700; 800; 900) for treatment of eye disorders, the eye patch comprising:
a first adhesive layer (410) configured for adhering an inner area (520; 620; 720; 820; 920) of the eye patch to secure an eyelid (20) of a user closed, the first adhesive layer comprising a silicone adhesive contact layer having a first coat formula;
a second adhesive layer (460) configured for adhering an outer area (510; 610; 710; 810; 910) of the eye patch to an area (10) around an eye of the user, the second adhesive layer comprising a silicone adhesive contact layer having a second coat formula, the second coat formula being of stronger adherence than the first coat formula of the first adhesive layer (410), the second adhesive area having first and second portions where the second portion is configured to surround the first portion and to be attached to the area (10) around the eye of the user;
a reflecting layer (440) adhered to the first portion of the second adhesive layer (460), the reflecting layer being configured for reflecting radiation towards the eyelid (20) of a user;
a flexible element (440; 530; 630; 730; 830; 930) positioned in the inner area (520; 620; 720; 820; 920) of the eye patch between the first layer (440) and the first adhesive layer (410) and configured for providing radiation therapy in accordance with a predetermined radiation therapy program; and
a connection (135a, 135b; 235a, 235b) configured for connecting the flexible element (440; 530; 630; 730; 830; 930) to a control unit (300) and for providing electrical power to the flexible element in accordance with the predetermined radiation therapy.

2. The eye patch of claim 1, wherein the flexible element (440; 530; 630; 730; 830; 930) comprises a heat coil configured for receiving electrical energy and for emitting radiant energy in response to the received electrical energy.

3. The eye patch of claim 2, wherein the heat coil is rated up to 1W.

4. The eye patch of claim 2 or 3, wherein the radiant energy comprises both thermal energy and visible light energy.

5. The eye patch of any one of claims 2 to 4, wherein the flexible element (440; 530; 630; 730; 830; 930) includes a layer of graphene.

6. The eye patch of any one of claims 1 to 5, wherein the reflecting layer comprises a layer of infrared reflective material.

7. The eye patch of claim 6, wherein the infrared reflective material comprises one of: a woven material and a non-woven material.

8. The eye patch of any one of claims 1 to 7, further comprising a backing layer (420) for the first adhesive layer (410), the backing layer being on a side of the first adhesive layer nearer to the second adhesive layer (460).

9. The eye patch of claim 8, further comprising a permanent adhesive layer (430) formed on the backing layer (420) between the first adhesive layer (410) and the flexible element (450) and the reflecting layer (440), the permanent adhesive layer being configured for retaining the flexible element in position with respect to the first adhesive layer, and, for retaining the reflecting layer.

10. The eye patch of any one of claims 1 to 9, further comprising a light pipe (640; 740; 840; 940) located around the flexible element (440; 530; 630; 730; 830; 930) between the first and second adhesive layers (410, 460), the connection (235a, 235b) forming a dual connection providing both electrical power to the flexible element (235) and light to light pipe.

11. The eye patch of claim 10, further comprising branches (840a, 840b, 840c) off the light pipe (840) configured for directing light therapy to areas around the eye.

12. The eye patch of any one of claims 1 to 10, further comprising a plurality of light-emitting diode elements (940a, 940b, 940c) configured to branch into the outer area (910) for directing light therapy to areas around the eye.

13. A treatment system (100; 200) comprising:
a headband (110) configured to be worn by a user, the headband including a front portion (110a) and a rear portion (110b) and the front portion includes a cavity (110c) formed therein;
a control unit (300) configured to be mounted in the cavity (110c) of the headband (110); and
at least one an eye patch of any one of claims 1 to 8, each eye patch being configured to be connected to the control unit (300) and stored within the cavity (110c) of the headband (110) when not in use.

14. The treatment system of claim 13, wherein the control unit (300) is configured to be programmable, and has at least one program for controlling one or more sequential therapy cycles.

15. The treatment system of claim 14, wherein the control unit (300) further includes a setting for individual eye treatment allowing one eye to function while the other eye is being treated.

## Patentansprüche

1. Augenklappe (400; 500; 600; 700; 800; 900) zur Behandlung von Augenbeschwerden, wobei die Augenklappe umfasst:
eine erste Haftschicht (410), welche zum Haften eines Innenbereichs (520; 620; 720; 820; 920) der Augenklappe konfiguriert ist, um ein Augenlid (20) eines Benutzers geschlossen zu halten, wobei die erste Haftschicht eine Silikon-Haftkontaktschicht umfasst, welche eine erste Beschichtungsformel aufweist;
eine zweite Haftschicht (460), welche zum Haften eines Außenbereichs (510; 610; 710; 810; 910) der Augenklappe an einem Bereich (10) rund um ein Auge des Benutzers konfiguriert ist, wobei die zweite Haftschicht eine Silikon-Haftkontaktschicht umfasst, welche eine zweite Beschichtungsformel aufweist; wobei die zweite Beschichtungsformel eine stärkere Haftung als die erste Beschichtungsformel der ersten Haftschicht (410) aufweist, wobei der zweite Haftbereich erste und zweite Abschnitte aufweist, wobei der zweite Abschnitt konfiguriert ist, um den ersten Abschnitt zu umgeben und an dem Bereich (10) rund um das Auge des Benutzers angebracht zu werden;
eine Reflexionsschicht (440), welche an dem ersten Abschnitt der zweiten Haftschicht (460) haftet, wobei die Reflexionsschicht zum Reflektieren von Strahlung zu dem Augenlid (20) eines Benutzers konfiguriert ist;
ein flexibles Element (440; 530; 630; 730; 830; 930), welches in dem Innenbereich (520; 620; 720; 820; 920) der Augenklappe zwischen der ersten Schicht (440) und der ersten Haftschicht (410) positioniert und zum Bereitstellen von Strahlungstherapie in Übereinstimmung mit einem vorbestimmten Strahlungstherapieprogramm konfiguriert ist; und
eine Verbindung (135a, 135b; 235a, 235b), welche zum Verbinden des flexiblen Elements (440; 530; 630; 730; 830; 930) mit einer Steuereinheit (300) und zum Bereitstellen elektrischer Leistung für das flexible Element in Übereinstimmung mit der vorbestimmten Strahlungstherapie konfiguriert ist.

2. Augenklappe nach Anspruch 1, wobei das flexible Element (440; 530; 630; 730; 830; 930) eine Hitzdrahtspule umfasst, welche zum Empfangen von elektrischer Energie und zum Abgeben von Strahlungsenergie als Reaktion auf die empfangene elektrische Energie konfiguriert ist.

3. Augenklappe nach Anspruch 2, wobei die Hitzdrahtspule einen Nennwert von bis zu 1 W aufweist.

4. Augenklappe nach Anspruch 2 oder 3, wobei die Strahlungsenergie sowohl thermische Energie als auch sichtbare Lichtenergie umfasst.

5. Augenklappe nach einem der Ansprüche 2 bis 4, wobei das flexible Element (440; 530; 630; 730; 830; 930) eine Schicht Graphen beinhaltet.

6. Augenklappe nach einem der Ansprüche 1 bis 5, wobei die Reflexionsschicht eine Schicht Infrarot-Reflexionsmaterial umfasst.

7. Augenklappe nach Anspruch 6, wobei das Infrarot-Reflexionsmaterial eines von einem Webstoff und einem Vliesstoff umfasst.

8. Augenklappe nach einem der Ansprüche 1 bis 7, weiter eine Trägerschicht (420) für die erste Haftschicht (410) umfassend, wobei die Trägerschicht sich an einer Seite der ersten Haftschicht näher an der zweiten Haftschicht (460) befindet.

9. Augenklappe nach Anspruch 8, weiter eine permanente Haftschicht (430) umfassend, welche auf der Trägerschicht (420) zwischen der ersten Haftschicht (410) und dem flexiblen Element (450) und der Reflexionsschicht (440) gebildet ist, wobei die permanente Haftschicht zum Halten des flexiblen Elements in Position in Bezug auf die erste Haftschicht und zum Halten der Reflexionsschicht konfiguriert ist.

10. Augenklappe nach einem der Ansprüche 1 bis 9, weiter einen Hohllichtleiter (640; 740; 840; 940) umfassend, welcher sich rund um das flexible Element (440; 530; 630; 730; 830; 930) zwischen der ersten und zweiten Haftschicht (410, 460) befindet, wobei die Verbindung (235a, 235b) eine duale Verbindung bildet, welche sowohl elektrische Leistung für das flexible Element (235) als auch Licht für den Hohllichtleiter bereitstellt.

11. Augenklappe nach Anspruch 10, weiter Abzweigungen (840a, 840b, 840c) von dem Hohllichtleiter (840) umfassend, welche zum Richten von Lichttherapie auf Bereiche rund um das Auge konfiguriert sind.

12. Augenklappe nach einem der Ansprüche 1 bis 10, weiter eine Vielzahl von Leuchtdiodenelementen (940a, 940b, 940c) umfassend, welche konfiguriert sind, um zum Richten von Lichttherapie auf Bereiche rund um das Auge in den Außenbereich (910) abzuzweigen.

13. Behandlungssystem (100; 200), umfassend:
ein Stirnband (110), welches konfiguriert ist, um von einem Benutzer getragen zu werden, wobei das Stirnband einen Vorderabschnitt (110a) und einen Hinterabschnitt (110b) beinhaltet und der Vorderabschnitt einen darin gebildeten Hohlraum (110c) beinhaltet;
eine Steuereinheit (300), welche konfiguriert ist, um in dem Hohlraum (110c) des Stirnbands (110) montiert zu werden; und
zumindest eine Augenklappe nach einem der Ansprüche 1 bis 8, wobei jede Augenklappe konfiguriert ist, um mit der Steuereinheit (300) verbunden und innerhalb des Hohlraums (110c) des Stirnbands (110) aufbewahrt zu werden, wenn nicht in Gebrauch.

14. Behandlungssystem nach Anspruch 13, wobei die Steuereinheit (300) konfiguriert ist, um programmierbar zu sein, und zumindest ein Programm zum Steuern eines oder mehrerer aufeinanderfolgender Therapiezyklen aufweist.

15. Behandlungssystem nach Anspruch 14, wobei die Steuereinheit (300) weiter eine Einstellung für individuelle Augenbehandlung beinhaltet, welche erlaubt, dass ein Auge funktioniert, während das andere Auge behandelt wird.

## Revendications

1. Pansement oculaire (400 ; 500; 600; 700; 800; 900) pour le traitement de troubles oculaires, le pansement oculaire comprenant :
une première couche adhésive (410) configurée pour faire adhérer une zone intérieure (520 ; 620 ; 720 ; 820 ; 920) du pansement oculaire pour fixer une paupière (20) d'un utilisateur fermée, la première couche adhésive comprenant une couche de contact adhésive en silicone présentant une première formule de couche ;
une seconde couche adhésive (460) configurée pour faire adhérer une zone extérieure (510 ; 610 ; 710 ; 810 ; 910) du pansement oculaire à une zone (10) autour d'un oeil de l'utilisateur, la seconde couche adhésive comprenant une couche de contact adhésive en silicone présentant une seconde formule de revêtement, la seconde formule de revêtement étant d'une adhérence plus forte que la première formule de revêtement de la première couche adhésive (410), la seconde zone adhésive présentant des première et seconde parties où la seconde partie est configurée pour entourer la première partie et pour être fixée à la zone (10) autour de l'œil de l'utilisateur ;
une couche réfléchissante (440) collée à la première partie de la seconde couche adhésive (460), la couche réfléchissante étant configurée pour réfléchir le rayonnement vers la paupière (20) d'un utilisateur ;
un élément flexible (440 ; 530; 630; 730; 830; 930) positionné dans la zone intérieure (520 ; 620 ; 720 ; 820 ; 920) du pansement oculaire entre la première couche (440) et la première couche adhésive (410) et configuré pour fournir une radiothérapie conformément à un programme de radiothérapie prédéterminé ; et
un raccord (135a, 135b ; 235a, 235b) configuré pour raccorder l'élément flexible (440 ; 530 ; 630 ; 730 ; 830 ; 930) à une unité de commande (300) et pour fournir une énergie électrique à l'élément flexible en fonction de la radiothérapie prédéterminée.

2. Pansement oculaire selon la revendication 1, dans lequel l'élément flexible (440 ; 530 ; 630 ; 730 ; 830 ; 930) comprend un serpentin thermique configuré pour recevoir une énergie électrique et pour émettre une énergie radiante en réponse à l'énergie électrique reçue.

3. Pansement oculaire selon la revendication 2, dans lequel le serpentin thermique est évalué à 1W maximum.

4. Pansement oculaire selon la revendication 2 ou 3, dans lequel l'énergie radiante comprend à la fois de l'énergie thermique et de l'énergie lumineuse visible.

5. Pansement oculaire selon l'une quelconque des revendications 2 à 4, dans lequel l'élément flexible (440 ; 530 ; 630 ; 730 ; 830 ; 930) inclut une couche de graphène.

6. Pansement oculaire selon l'une quelconque des revendications 1 à 5, dans lequel la couche réfléchissante comprend une couche de matériau réfléchissant les infrarouges.

7. Pansement oculaire selon la revendication 6, dans lequel le matériau réfléchissant les infrarouges comprend l'un des éléments suivants : un matériau tissé et un matériau non tissé.

8. Pansement oculaire selon l'une quelconque des revendications 1 à 7, comprenant en outre une couche de support (420) pour la première couche adhésive (410), la couche de support étant sur un côté de la première couche adhésive plus proche de la seconde couche adhésive (460).

9. Pansement oculaire selon la revendication 8, comprenant en outre une couche adhésive permanente (430) formée sur la couche de support (420) entre la première couche adhésive (410) et l'élément flexible (450) et la couche réfléchissante (440), la couche adhésive permanente étant configurée pour retenir l'élément flexible en position par rapport à la première couche adhésive, et pour retenir la couche réfléchissante.

10. Pansement oculaire selon l'une quelconque des revendications 1 à 9, comprenant en outre un conduit de lumière (640; 740; 840; 940) situé autour de l'élément flexible (440 ; 530; 630; 730; 830; 930) entre les première et seconde couches adhésives (410, 460), le raccord (235a, 235b) formant un double raccord fournissant à la fois de l'énergie électrique à l'élément flexible (235) et de la lumière au conduit de lumière.

11. Pansement oculaire selon la revendication 10, comprenant en outre des branches (840a, 840b, 840c) du conduit de lumière (840) configurées pour diriger la luminothérapie vers des zones autour de l'œil.

12. Pansement oculaire selon l'une quelconque des revendications 1 à 10, comprenant en outre une pluralité d'éléments de diodes électroluminescentes (940a, 940b, 940c) configurés pour se ramifier dans la zone extérieure (910) pour diriger la luminothérapie vers des zones autour de l'œil.

13. Système de traitement (100 ; 200) comprenant :
un bandeau (110) configuré pour être porté par un utilisateur, le bandeau incluant une partie avant (110a) et une partie arrière (110b) et la partie avant incluant une cavité (110c) formée dans celle-ci ;
une unité de commande (300) configurée pour être montée dans la cavité (110c) du bandeau (110) ; et
au moins un pansement oculaire selon l'une quelconque des revendications 1 à 8, chaque pansement oculaire étant configuré pour être raccordé à l'unité de commande (300) et stocké au sein de la cavité (110c) du bandeau (110) lorsqu'il n'est pas utilisé.

14. Système de traitement selon la revendication 13, dans lequel l'unité de commande (300) est configurée pour pouvoir être programmée, et présente au moins un programme pour commander un ou plusieurs cycles thérapeutiques séquentiels.

15. Système de traitement selon la revendication 14, dans lequel l'unité de commande (300) inclut en outre un réglage pour le traitement individuel des yeux permettant à un oeil de remplir sa fonction pendant que l'autre oeil est traité.
